# EUROPEAN PATENT SPECIFICATION

(11) **EP 1 718 290 B1**
(45) Date of publication and mention of the grant of the patent: **05.05.2010**
(21) Application number: 05708155.6
(22) Date of filing: 04.02.2005
(51) Int. Cl.: A61K 31/198, A61K 33/00, A61K 31/519, A61P 17/06

(54) **COMPOSITION, COMPRISING L-SERINE, L-ISOLEUCINE, FOLIC ACID AND TRACE ELEMENTS, FOR TREATING PSORIASIS**
ZUSAMMENSETZUNG MIT L-SERIN, L-ISOLEUCIN, FOLSÄURE UND SPURENELEMENTEN ZUR BEHANDLUNG VON PSORIASIS
COMPOSITION A BASE DE L-SERINE, DE L-ISOLEUCINE, D'ACIDE FOLIQUE ET D'OLIGO-ELEMENTS, UTILISEE DANS LE TRAITEMENT DU PSORIASIS

(30) Priority: 06.02.2004 FI 20040180
(43) Date of publication of application: 08.11.2006
(73) Proprietor: Oy Neurofood Ab, 02270 Espoo (FI)
(72) Inventor: TALLBERG, Thomas, FI-00200 Helsingfors (FI)
(74) Representative: Boije-Backman, Solveig Magdalena
(86) International application number: PCT/FI2005/000075
(87) International publication number: WO 2005/074910

(56) References cited:
- WO-A1-89/01930
- GB-A- 2 370 504
- US-A- 5 006 337
- US-A1- 2004 086 583
- TALLBERG T. ET AL: 'Development of a Combined Biological and Immunological Cancer Therapy Modality' JOURNAL OF AUSTRALIAN COLLEGA OF NUTRITIONAL AND EVIRONMENTAL MEDICINE vol. 22, no. 1, 01 April 2003, pages 3 - 21, XP002987380
- S.R.L.A. SALO: 'Composizione Per Uso Cosmetico o Farmaceutico A Base Di Acido Lipoico E Acido Piruvico' MINISTERO DELL INDUSTRIA DEL COMMERCIO E DELL ARTIGIANATO 24 March 2000,
- TALLBERG T. ET AL: 'Studies on Mitochondrial Regulation of the Genome' DEUTSCHE ZEITSCHRIFT FUER ONKOLOGIE vol. 34, 2002, pages 128 - 139, XP002987381

## Description

### Field of the Invention

The present invention relates to the use of natural biological components for the manufacture of a pharmaceutical composition for treating or preventing psoriasis. The invention further relates to a pharmaceutical composition comprising the natural biological components as sole pharmaceutically active ingredients. This composition has a curing effect on the skin disease.

### Background of the Invention

Psoriasis is a skin disease of many varieties, characterized by the formation of scaly red patches on the extensor surfaces of the body. It is usually a chronic ailment, which seems to be linked to a certain genetic susceptibility. The treatment of these patients is usually complicated and expensive, although curative results are not regularly achieved. When the disease becomes progressive, it may also involve joints, and cause severe pain and restriction of the patients' mobility. Standard therapies have lately not changed appreciably, and the usual remedies are still light therapy, certain ointments and cortisone. No truly effective natural remedies have been devised. There is a great demand for improved methods for treating psoriasis, which methods should be effective, simple, inexpensive, and without harmful side effects.

Cancer is one of the major causes of death in the modem world, and great resources have been spent on finding remedy for this disease group. Conventionally cancer is treated by surgery, irradiation or with cytostatic agents. Alternative methods of treatment include for example a combined biological and immunological treatment modality called bio-immunotherapy (Tallberg, Thomas. Development of a Combined Biological and Immunological Cancer Therapy Modality. A review of Bio-Immunotherapy. J. Austr. Coll. Nutr. & Env. Med. 2003:22 p. 3-21). In bio-immunotherapy cancer patients are given natural biological components, which consist of a mixture of amino acids and trace elements, optionally in combination with neurogenic lipids and/or vitamins. Varying mixtures of these ingredients have been tested for different forms of cancer. The mixtures are preferably given as a food supplement.

The present invention is based on the surprising finding that during the treatment of cancer patients by bio-immunotherapy, it became apparent, with several cancer patients who by chance also suffered from psoriasis that their skin lesions disappeared, or the symptoms of the skin ailment were appreciably mitigated, although the cancer therapy they received was not directed against their skin disease. The positive effect on psoriasis seemed to be independent of the malignant disease, and was not linked to any form of cancer the patient was suffering from.

The present invention now provides the use of natural, biological components for the manufacture of a pharmaceutical composition for treating or preventing psoriasis.

The present invention further provides an inexpensive and safe pharmaceutical composition comprising natural biological components for treating or preventing psoriasis.

The invention also provides an alternative method of treating or preventing psoriasis by administering natural, biological components to a subject in need thereof.

### Short Description of the Invention

The invention relates to the use of L-amino acids serine (Ser) and isoleucine (Ile) for the manufacture of a pharmaceutical composition for treating or preventing psoriasis. Said amino acid is present in unbound form (previously called mono amino acid), which means that the amino acid is not included in a peptide or protein or any other macromolecule or conjugate, but it may be in the form of a complex with the trace element ions of the composition.

The invention further relates to a pharmaceutical composition comprising L-amino acids serine (Ser) and isoleucine (Ile), optionally combined with at least one trace element selected from the group consisting of chromium (Cr), tin (Sn), selenium (Se), vanadium (V) and wolfram (W), and/or with folic acid as sole pharmaceutically active ingredients.

A method of treating or preventing psoriasis by administering a pharmaceutically active amount of L-amino acids serine (Ser) and isoleucine (Ile) to a subject in need thereof is also disclosed.

Some preferred embodiments of the invention are set forth in the dependent claims.

### Detailed Description of the Invention

Upon analysis on which of the natural biological components the cancer patients with psoriasis had ingested, it became evident that certain amino acids together with specific essential trace-element metal ions used in the bio-immunotherapy they received were responsible for the positive clinical effect on their skin disease. In close family members of these cancer patients, who otherwise were healthy but who also had suffered from bouts of psoriasis, the same natural dietary components ingested could cause a favorable clinical effect.

Psoriasis patients who were not suffering from cancer were therefore studied. In numerous cases tested the active dietary components have been delineated. The oral intake prescribed, in the form of a food supplementation to these patients is based on a formulation comprising a combination of the amino acids isoleucine (Ile) and serine (Ser). Said two amino acids are administered in the form of L-amino acids.

In addition to Ile and Ser the composition preferably also comprises at least one of the essential trace elements selected from the group consisting of chromium (Cr), tin (Sn), selenium (Se), vanadium (V) wolfram (W) and zinc (Zn). Optionally the trace elements also include e.g. manganese (Mn). To be biologically active the trace elements shall be in ionic form, wherefore they in practice are administered as salts. A salt having a neutral taste is preferred, and salts having a strong or bad taste should be avoided.

Preferably the formulation for treating or preventing psoriasis further comprises physiologic amounts of folic acid.

The treatment successfully used according to the present invention is based on the oral administration of L-serine and L-isoleucine, optionally together with essential trace elements and folic acid as active ingredients. According to one embodiment of the invention the composition consists essentially of said three groups of components i.e. the amino acids, the trace elements and the vitamin. All these components occur in nature and are biologically active, which means that they take part in biological events such as metabolic processes. Very good results were obtained with a composition comprising L-serine, L-isoleucine, salts of Cr, Sn, Se, V and W, and folic acid as sole pharmaceutically active ingredients.

The composition used comprises the components in biologically and pharmaceutically active amounts that is amounts sufficient to achieve the desired health promoting effect. As will be readily understood by a physician, the amounts will vary depending on the individual and his health status, as well as other factors such as weight, age, nutrition, stress and environmental factors, etc. Examples of suitable amounts include, but are not limited to, about 2 - 10, usually 2 - 5 g/day of each of L-serine and L-isoleucine. The trace element are usually used in amounts of 0.5 - 9 mg/day, preferably 1 - 3 mg/day of each of the trace element ions of Cr, Sn, Se, V and W. Zn may be administered in doses of about 15 mg/day and Mn in doses of about 50 mg/day. The folic acid is used in small, well-established physiological amounts, such as 1 - 2 mg/day.

In order to improve lymphopoiesis and the immunedefence in the patients prion-free neurogenic lipids may also be administered to the patients being treated with the above disclosed composition. Neurogen lipids are obtainable e.g. from the brain of healthy young pigs. The brain tissue is boiled, frozen and lyophilised, whereafter the lipids are extracted with ether-ethyl alcohol as previously described to obtain a prion-free lipid fraction (Tallberg T. et al. Cancer Immunity. The Effect in Cancer-Immunotherapy of Polymerised Autologous Tumour Tissue and Supportive Measures. Scand. J. Lab. Invets. 1979:39 p. 3-33). Neurogenic lipids are purchased and canned by Neurofood Ltd., Finland. The recommended daily intake of neurogenic lipids is equivalent to about 50 - 100 g brain.

The ingredients of the composition including amino acids, trace elements and vitamin can be administered as such either separately or in varying combinations. They may be purchased e.g. in powder form separately, or as ready made powders containing all ingredients. Such a powder mixture may be pre-packed and used as such or as a supplement to conventional food items e.g. in the patient's morning yoghurt. For the consumer it is easy to ingest in connection with breakfast or as a snack between meals. This compensatory dietary treatment is comparatively inexpensive, since it consists of only natural components, forming the active food additive. Of course the pharmaceutical composition may also be processed into granulates, capsules or tablets, which may comprise pharmaceutically acceptable carriers or excipients. The neurogenic lipid product can be administered either simultaneously with the other ingredients or separately at a different time. A preferred way is to mix the neurogenic lipid product in assorted fruits and ingest it chilled.

The invention is illustrated by the following example, which should not be construed as restricting its scope in any way. A person skilled in the art will be able to make variations and modifications thereof, having the same beneficial effects as described here.

### Example

Psoriasis patients, who were not suffering from cancer were given orally in the form of a food supplementation isoleucine and serine together with milligram amounts of certain biologically active trace-element salts, in which the metal ions are present in a biologically active form. Some milligrams of folic acid were also included in the composition. This natural food supplementation was given to the patients every day. The dietary bio-modulation schedule for treatment of patients suffering from psoriasis was as follows:

### Combined supportive dietary measures:

1. Oral administration of (2 - 5 g/day) of each of the respective L-amino acids; serine (Ser), and isoleusine (Ile), in connection with meals.
2. Essential trace-element salts orally as biologically active ions, at dose levels of some milligrams (1-3 mg/day); chromium (CrCl₃ · 6 H₂O) 6mg (= 1.17 mg Cr), tin (SnCl4 · 5 H₂O) 4mg (= 1.35mg Sn), selenium Na₂SeO₄ · 10 H₂O, 6mg (= 1.28 mg Se), vanadium (Na₂VO₄ · 4 H₂O), 6mg (= 2.5 mg V), wolfram (Na₂WO₄ · 2 H₂O), 4mg (= 2.3mg W). Some patients also received zinc gluconate in daily amounts corresponding to 15 mg Zn ions, and some received manganese sulphate in amounts of 140 mg/day (= 51.0 mg Mn).
3. Small physiologic amounts of folic acid (1-2 mg/day).
4. Some of the patients received a diet additionally containing prion-free neurogenic lipids equivalent to approx. 50 g of brain (purchased and canned by Neurofood Ltd. Finland).
5. The patients received pre-packed powders comprising a mixture of ingredients 1 - 3, and were recommended to mix the powder into their morning yoghurt to get their daily ration.
6. Dose-levels were adjusted based on clinical response observed, and correlated to the patients' body weight.

The positive effect of the treatment in over ten patients tested was seen in some months time. Locally the skin patches became less irritating and some disappeared completely. Joint pain subsided. The effect lasted up to 20 years in some cases under continuous administration of the composition. The positive clinical outcome showed slight individual differences pertaining to the quantity and relative content of these biological ingredients, and the patients' body weight. In certain cases Ile alone combined with the trace element salts could cause a certain positive effect, but in other patients Ser seemed to be a necessary co-factor. This variation may be linked to certain genetic traits in patients suffering from psoriasis. This dietary treatment, aimed to compensate a metabolic deficiency underling this disease, was not linked to any side effects experienced in any of the voluntaries tested. This food additive can exert a lasting mitigating effect on patients suffering from psoriasis.

## Claims

1. A pharmaceutical composition consisting of:
a) 2 - 5 g of each L-amino acid serine (Ser) and isoleucine (Ile) in unbound form,
b) 1 - 3 mg of each chromium (Cr), tin (Sn), selenium (Se), vanadium (V), and wolfram (W) salts,
c) 1 - 2 mg of folic acid, and optionally one or more of the following zinc (Zn), manganese (Mn) and neurogenic lipids.

2. The pharmaceutical composition according to claim 1, wherein the composition is in powder form.

3. The pharmaceutical composition of any of the claims 1 - 2, wherein the composition is in the form of a dietary supplement.

4. The pharmaceutical composition of any of the claims 1-3, wherein the composition is for use in treatment and prevention of psoriasis.

## Patentansprüche

1. Pharmazeutische Zusammensetzung bestehend aus:
a) jeweils 2 bis 5 g L-Aminosäure-Serin (Ser) und Isoleucin (Ile) in ungebundener Form
b) jeweils 1 bis 3 mg Chrom- (Cr), Zinn- (Sn), Selen- (Se), Vanadium- (V) und Wolfram-Salz (W),
c) 1 bis 2 mg Folsäure und gegebenenfalls einem oder mehreren der folgenden: Zink (Zn), Mangan (Mn) und neurogene Lipide.

2. Pharmazeutische Zusammensetzung nach Anspruch 1, worin die Zusammensetzung in Pulverform vorliegt.

3. Pharmazeutische Zusammensetzung nach einem der Ansprüche 1 bis 2, worin die Zusammensetzung in Form eines Nahrungsergänzungsmittels vorliegt.

4. Pharmazeutische Zusammensetzung nach einem der Ansprüche 1 bis 3, worin die Zusammensetzung zur Behandlung und Vorbeugung von Psoriasis verwendet wird.

## Revendications

1. Composition pharmaceutique consistant en :
a) 2 à 5 g de chaque L-amino acide parmi la sérine (Ser) et l'isoleucine (Ile) sous forme non liée,
b) 1 à 3 mg de chacun des sels de chrome (Cr), d'étain (Sn), de sélénium (Se), de vanadium (V), et de tungstène,
c) 1 à 2 mg d'acide folique, et de manière optionnelle un ou plusieurs des éléments parmi le zinc (Zn), le manganèse (Mn) et des lipides neurogéniques.

2. Composition pharmaceutique selon la revendication 1, **caractérisée en ce que** la composition est sous forme de poudre.

3. Composition pharmaceutique selon l'une quelconque des revendications 1 à 2, **caractérisée en ce que** la composition est sous forme de supplément diététique.

4. Composition pharmaceutique selon l'une quelconque des revendications 1 à 3, **caractérisée en ce que** la composition est utilisée pour le traitement et la prévention du psoriasis.
